# EUROPEAN PATENT SPECIFICATION

(11) **EP 2 793 970 B1**
(45) Date of publication and mention of the grant of the patent: **16.11.2016**
(21) Application number: 12859392.8
(22) Date of filing: 20.12.2012
(51) Int. Cl.: A61M 1/00, A61M 39/10

(54) **ADJUSTABLE SUCTION TIPS FOR DENTAL AND MEDICAL USES**
EINSTELLBARE SAUGSPITZEN FÜR DENTALE UND MEDIZINISCHE VERWENDUNGEN
POINTES D'ASPIRATION AJUSTABLES POUR UTILISATIONS DENTAIRES ET MÉDICALES

(30) Priority: 23.12.2011 US 201113336403
(43) Date of publication of application: 29.10.2014
(73) Proprietor: GSN Products, Inc., Glendale, CA 91202 (US)
(72) Inventor: KARAPETIAN, Hovik, Montrose, CA 91020 (US); KHOJASARIAN, Saro, Glendale, CA 91201 (US); ODABASHIAN, Nishan, Glendale, CA 91202 (US)
(74) Representative: Brizio Delaporte, Allison
(86) International application number: PCT/US2012/070976
(87) International publication number: WO 2013/096634

(56) References cited:
- EP-A1- 0 390 951
- DE-U1-202010 009 610
- GB-A- 439 542
- US-A- 2 519 595
- US-A- 5 116 223
- US-A- 5 645 539
- US-A- 5 743 736
- US-A- 6 022 326
- US-A1- 2005 107 671
- US-A1- 2005 137 581
- US-A1- 2010 249 755
- US-B1- 6 419 414

## Description

### RELATED APPLICATIONS

This application claims priority as a continuation-in-part to commonly-owned US application Ser. No. 13/336,403, filed December 23, 2011 and entitled the same.

### GOVERNMENT RIGHTS

None.

### TECHNICAL FIELD

The invention relates to aspirating devices in the medical, dental and veterinary arts, but with potential application to other fields as well.

### BACKGROUND ART

Dentistry, oral surgery and medical procedures in general typically require aspiration of liquid and debris, and/or retraction of soft tissues. This historically has been accomplished using straight or fixed-angled "evacuator" suction tips. The fixed nature and relatively high-volume/speed of these contributes to an overall inefficient and uncomfortable process. For example, surfaces to be suctioned/retracted are often of numerous different presentations and angles, and often in cramped, confined spaces affording limited operator vision. Using a straight or fixed-tip implement leads to tedious, inefficient and incomplete contacting, with consequent inefficient suctioning and retraction. This in turn protracts and complicates procedures, often working discomfort on both patient and operator alike. It is estimated that this occurs millions of times each day around the world, and has been a problem ever since the advent of modem medicine and dentistry.

A cousin of the evacuator is the saliva ejector. It typically has a relatively flexible, arcuate or "u-shape" design that permits it to passively hang from a patient's mouth. It typically also has a narrower internal lumen space at the point of attachment with a vacuum/suction hose. (~0.25"/0.635 cm diameter versus ~0.50"/1.27 cm diameter for evacuators). This, coupled with a bulbous venting screen over its contacting end, promotes facilitates liquid passage while simultaneously impeding solid passage and diffusing suction force away from soft tissue. Because of its passive nature, the saliva ejector also does not have a "handle" in the typical sense that an evacuator does.

The differences between evacuator tips and saliva ejectors are therefore a function of their respective specialized uses. Not surprisingly, the need for these different uses often present together during the same procedures. Because of this, each tip type is typically present and attached to a dedicated hose on a common multi-hose pump assembly standard in most dental, medical, and surgical settings.

The following documents illustrate some of the drawbacks with existing aspiration tips prior to the invention: US Patent 5,061,180 to Gary B. Wiele, filed for on May 1, 1989, and issued on October 29, 1991 (describing a combined vacuum/irrigation implement made out of fitted concentric tube pieces that afford internal rotation and longitudinal sliding of conduits within but limited or no lateral adjustability); US Patent 4,883,426 to Euler Ferrer, filed for on July 5, 1988 and issued on November 28, 1999 (disclosing a fixed arcuate-shaped tissue-retraction and suction tip featuring a lateral orifice that permits modulated suction); US Patent 4,878,900 to T.M Sunt, issued in November, 1989 (teaching similarly); US Patent 3,090,122 to N.R. Erickson, filed for on January 18, 1961 and issued on May 21, 1963 (teaching similarly); US 2010/0203470 A1 to Sidhu and Behoteguy, published August 12, 2010 (teaching similarly but with straight evacuator and capture screen).

Various attempts have been described to overcome these drawbacks but have so far not met with success, i.e., they have proven overly complicated, expensive, impractical and/or unworkable. Some are typified by joint-less, flexible hose portions made from plastic incorporating malleable wiring and/or wire ribbing *See, e.g.,* US Patent 4,487,600 to A.W. Brownlie et al., filed for on May 1, 1989, and issued on October 29, 1991; US Patent 3256885 to J.L. Higgens et al., filed for on June 26, 1963 and issued on June 21, 1966. Another attempt, illustrated in US Patent 5,743,736 to M. Folko et al., filed for on November 3, 1994 and issued on April 28, 1998, features a cumbersome multi-jointed saliva ejector, including a bulbous suction screen head.

Evacuators in particular remain relatively inflexible and inefficient in adapting to cover different surfaces and angles. Nor are they ergonomic or optimum in promoting and retaining line of site during procedures. There remains the need for a simple, inexpensive, adjustable implement of greater versatility that would ameliorate one or more of the above long-felt, unsolved art needs.

### SUMMARY OF THE INVENTION

### TECHNICAL PROBLEM

Medical and dental evacuators have long suffered ergonomic, line-of-sight, and inefficient suction-surface interfacing challenges.

### SOLUTION TO PROBLEM

It is an object of the invention to address one or more of these historical challenges by providing an adjustable, joint-based implement. The implement is additionally useful in that it is relatively simple in design, easy and versatile in use, cheap to construct, and lends well to disposability and hygiene.

The adjustable suctioning implement according to the invention is defined such as in claim 1. A kit according to the invention is defined such as in claim 11. A method of using the adjustable suctioning implement according to the invention is defined such as in claim 15.

In a first aspect the invention features an adjustable suctioning implement, preferably an evacuator for dental or medical applications. The implement includes a hollow handle portion having a source end for connecting to a vacuum line or pump and a second end. The implement also features a hollow nozzle portion having a contacting end for engaging a patient surface to be suctioned and a second end for functionally engaging, directly or indirectly, the second end of the handle portion. The implement further includes a hollow joint that is at least partially defined by the second end of the handle and the second end of the nozzle. The implement can be fashioned out of two or more distinct pieces but preferably contains no more than three (including an adaptor), the latter of which is preferably configured in a dual opposing ball or socket configuration. In preferred embodiments, the source end of the handle is configured to mate with an evacuator hose line, preferably one on a multi-hose pump assembly, wherein are present a plurality of different diameter suction hose lines. Evacuator lines typically fall within the range of about 0.40 and 0.60 inches (1.016-1.524 cm), with about 0.5 inches/1.27 cm most typical.

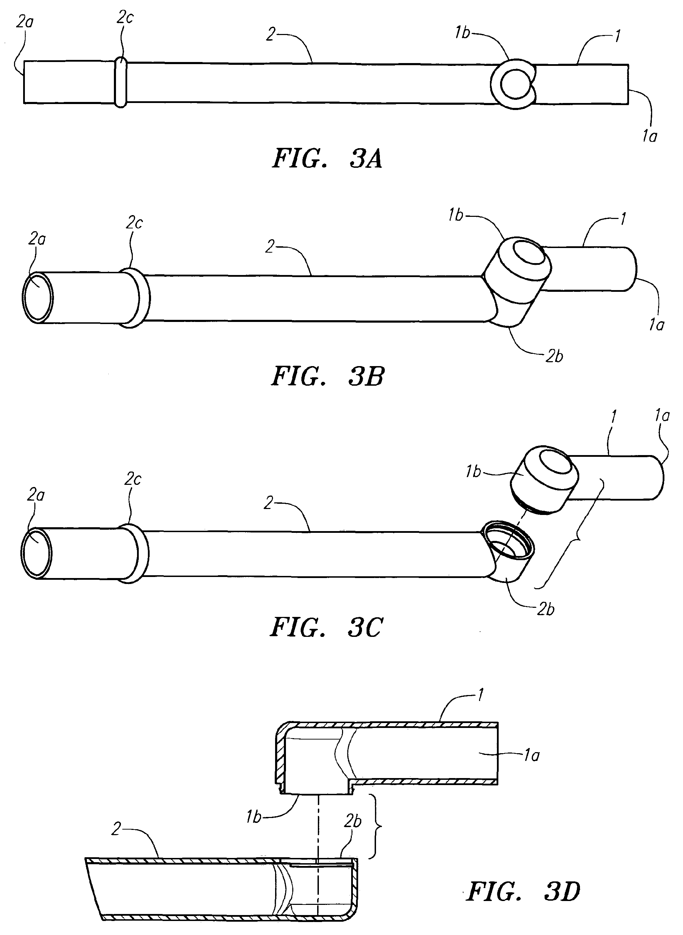

Although the joint according to the invention such as claimed features two balls and sockets, in not claimed examples the joint features one ball and socket, or in another so-called "rotary" embodiment the joint can feature a hollow pivoting drum, e.g., as illustrated in the Figure 3, one end of which preferably is contiguous with or integral to the handle and the other end of which preferably is contiguous with or integral to the nozzle. The drum (and for that matter, the handle, nozzle, and joint) can be any dimension, e.g., cylindrical, barrel-shaped, rectangular, square, triangular, elliptical, etc. as long as the portion thereof that interfaces cooperatively with the handle allows rotation about an axis perpendicular or substantially perpendicular thereto.

Additional aspects of the invention feature kits and methods of using the implements of the first aspect.

Various embodiments are discussed in the detailed description and accompanying drawings and claims, and yet additional embodiments will be immediately apparent to the person of ordinary skill in the art. For example, it will be appreciated that the invention may also find merit in other uses, e.g., automotive, marine, and manufacturing or servicing. It will also be appreciated that the invention could be alternatively or additionally configured to support irrigation and delivery techniques.

Any combination of individual embodiment features that are discussed can also be combined as suitable.

### ADVANTAGEOUS EFFECTS OF INVENTION

The invention ameliorates line-of-sight, ergonomic, and interfacing inefficiencies during medical and dental evacuation procedures.

### BRIEF DESCRIPTION OF DRAWINGS

Figures 1A-C show an embodiment not forming part of the invention such as claimed, featuring a single ball and socket joint as
   between handle and nozzle, with Figure 1A representing a side perspective view of the assembled implement, Figure 1B representing an exploded view of the same, and Figure 1C being a sectional view of the same showing potential for movement/adjustability about the joint.
Figures 2A-D show an embodiment according to the invention such as claimed, featuring a dual ball and socket joint as
   between handle and nozzle mediated by an intervening modular connector, with Figure 2A representing a side perspective view of the assembled implement, Figure 2B representing an exploded view of the same, and Figures 2C and 2D representing sectional views of the same.
Figures 3A-D show a rotary drum embodiment, not forming part of the invention such as claimed, with Figure 3A representing a top
   orthogonal view, Figure 3B representing a side perspective view, Figure 3C being an exploded perspective view, and Figure 3D representing a partial exploded sectional view of the joint from another angle.
Figures 4A-D show another rotary drum embodiment, not forming part of the invention such as claimed, with Figure 4A representing
   a top orthogonal view, Figure 4B representing a side perspective view, Figure 4C being an exploded perspective view, and Figure 4D representing a partial exploded sectional view of the joint from another angle.
Figure 5 shows one possible kit embodiment, wherein the tips are supplied in bulk and in straightened configuration to facilitate condensed bulk packaging.

### DESCRIPTION OF EMBODIMENTS

The implements of the invention, depending on aspect and embodiment, contemplate suction, irrigation and/or retraction using all types of fluids and dimensionally cooperative substances and tissues. This section governs only preferred aspects and embodiments of the invention. The person of ordinary skill in the art will, of course, appreciate that other aspects and embodiments are also well within the scope of the
invention, which is defined by the claims.

In medical and dental aspects and embodiments, body fluids include, e.g., blood and saliva. Irrigation fluids include, e.g., saline, distilled water, sodium hypochlorite, and chlorohexidine.

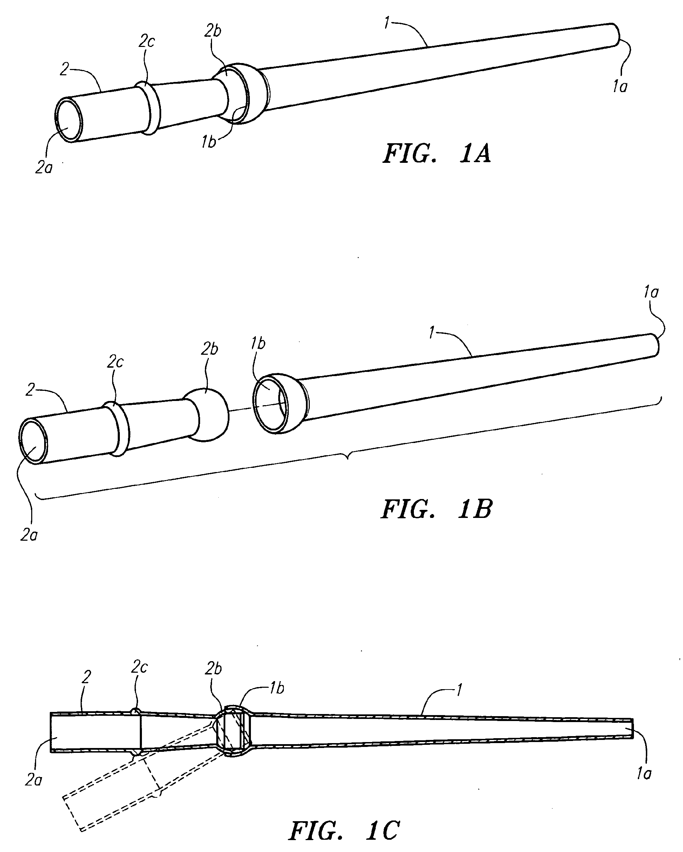

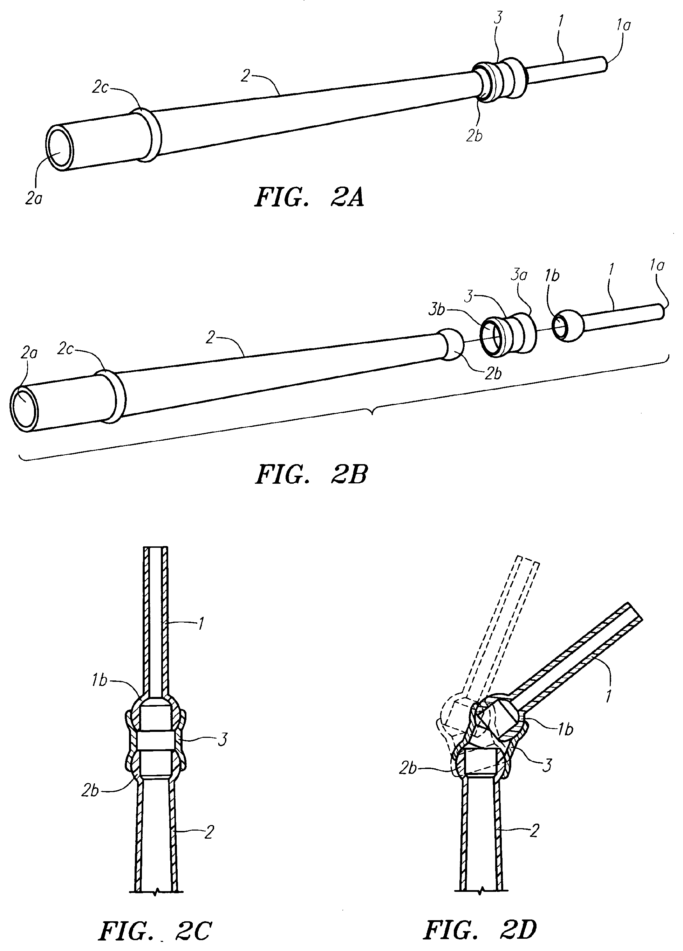

In most embodiments, the implement is assembled from multiple different pieces that functionally fit together at a joint. One preferred embodiment for this is a ball and socket design, in which a first piece bearing a ball-shaped end snaps snugly into the socket-like end of a second piece to allow articulation of the nozzle about the handle via the ball and socket joint between. Although the socket need not be perfectly conforming to the shape of the ball, it should be sufficiently tight that the vacuum/aspirating force within is substantially preserved during use yet the joint can still be manipulated upon sufficient application of force to adjust the angle of approach and/or contact. The advantage is that the joint is potentially movable in a variety of directions, thus endowing good adjustability. Configurations of the ball and socket can be as shown in the drawings or assume the reverse configuration, e.g., where the ball takes the place of the socket and vice-versa relative to the direction of air or fluid flow. Figure 1 and Figure 2 feature just two of many different possible ball and socket configurations that can be used, with the dual configuration shown in Figure 2 being according to the invention such as claimed and being preferred because of its greater range of motion.

As used herein, the term "joint" includes a single or dual ball and socket configuration, as opposed to the plural term "joints," which includes more than one such joint or multiple different types of joints. Also as used herein and throughout, and unless otherwise specified, the terms "medical," "dental", and "surgical" each embrace veterinary as well as human applications.

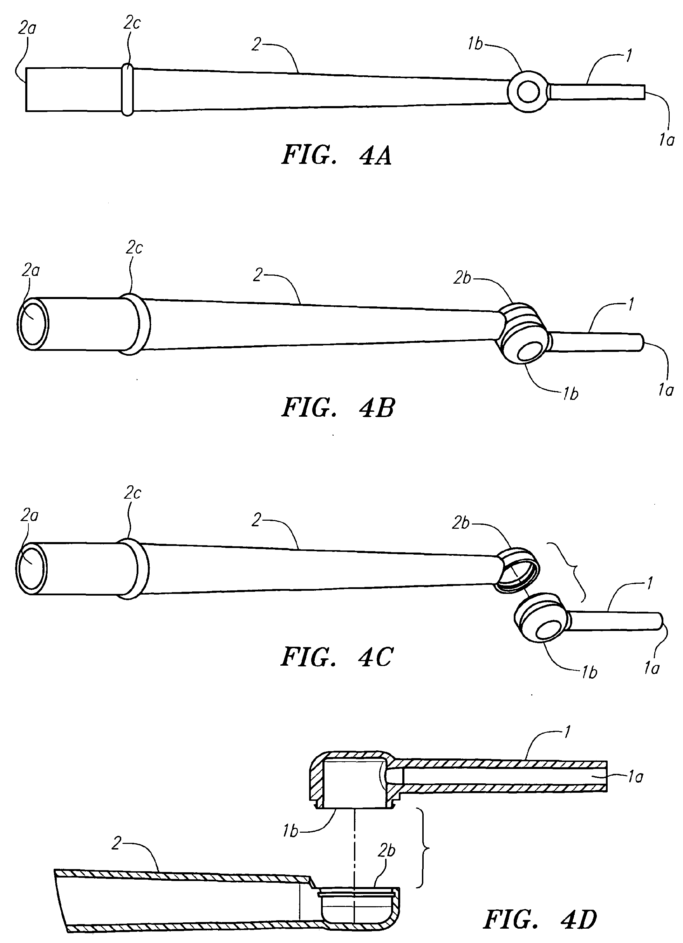

Another joint contemplated but not forming part of the invention such as claimed, features a rotary joint, two preferred
embodiments of which are depicted in Figures 3 and 4. This particular joint can assume a rotating drum configuration, or else be ball-and-socket configured as above, with the principal difference being that the ball and socket joint here is configured relatively perpendicular to the longitudinal axes of the handle and nozzle portions in Figures 1 and 2.

Implements of the invention can be made from one or more materials selected from the group consisting of metal, molded plastic, rubber, glass and paper, according to well-known methods. For many embodiments, plastic is preferred. Illustrative plastics that can be used include, e.g., polyvinyl chloride (PVC), polystyrene, polypropylene, polycarbonate, and acetate copolymers and these can be formed, e.g., by injection- or blow-molding techniques.

One or more of the handle, nozzle, and joint portions can be fashioned of different materials relative to the other portions. Any given portion can also be manufactured out of combinations of different materials as compatible/appropriate.

In some preferred embodiments where the handle is relatively straight, the joint allows rotational and/or angular pivoting about a central longitudinal axis defined by the handle. In other embodiments, the rotational and/or angular pivoting is offset relative to the aforementioned handle axis in a manner parallel or substantially parallel thereto, e.g., as a consequence of the mating configuration between handle and nozzle.

Each implement is hollow, with an external housing that defines at least one luminal space therein and there through, from one terminal end to the other. It is anticipated that one or more internal flexible tubes can also be fitted within the housing to accomplish the same and/or additional functions.

Joints can be fashioned using an independent connector module or else be formed entirely out of complementary integral ends of the nozzle and handle/body pieces.

In some preferred embodiments, the nozzle end is tapered or narrowly graded toward the suctioning end, which tapering or grading may or may not occur on the handle portion as well.

In some preferred embodiments, the suctioning end of the nozzle is also beveled and may also contain a screen spanning or proximate thereto and/or to the other end of the nozzle or implement to filter out and/or capture solids. However, most preferred embodiments feature a screen-less nozzle head.

In some preferred embodiments, colorants may be included for color-coding purposes. In other, not necessarily exclusive, embodiments the implement or one or more conduit portions thereof may be transparent or translucent.

In still further embodiments, fiber-optics or other lighting materials and materials for transmitting light can be incorporated into or alongside the plastic, as can malleable metallic wires or ribbing.

Some embodiments may also employ corrugation within the nozzle and/or handle portion to afford additional flexibility.

In some embodiments the implements are each sterilized and/or individually wrapped to promote hygiene. In other embodiments, the implements are reusable, e.g., via steam-autoclaving, infra-red irradiation or immersion in an antiseptic solution.

The implements of the invention, including their individual handle, nozzle and joint portions, can also be of different lengths, widths, diameters, wall thicknesses, tapering dimensions, colors and transparency/trans luminescence as appropriate for the particular type of procedure in which they are to be used. The term "handle" does not foreclose the use of a hand or finger on the mesial, facing or nozzle portion of the implement during use.

Preferred dimension ranges include 0.1-3.0 inches (0.254-7.62 cm) for nozzle length; nozzle width: 0.1-0.5 inches (0.254-1.27 cm); handle length: 1.0-6.0 inches (2.54-15.24 cm); handle width: 0.1-1.0 inches (0.254-2.54 cm); and wall thickness: 0.01-0.50 inches (0.0254-1.27 cm).

The implements of the invention may also feature a flanged exterior ring on the handle portion that services as a sealing means between vacuum/pump-line and handle. The line essentially slips over a portion of the handle and seals against the ring. This ring is typically located about 0.5-1.0 inches (1.27-2.54cm) from the source end of the handle.

The implements may also feature one or more laterally positioned apertures, vents, or ports located along the side housing of the nozzle or handle and that are in communication with the internal lumen/vacuum space of the implement. These can be more or less covered by the operator's finger(s) or by slide-able or depressible flaps actuated by such during use, thereby providing for modulation of the amount of suction or positive force exerted at the contact point during a procedure, be it suctioning, blowing or irrigation.

Another way to modulate pressure or increase accessibility is to create a modified vacuuming surface at the point of engagement, e.g., by supply of a nozzle facing that is not perpendicular to the length of the nozzle but cut or molded at an angle thereto.

In some embodiments the contacting end can also be closed as opposed to open and suction/evacuation/irrigation is accomplished using one or more lateral holes, ports or apertures, typically smaller in dimension and distinct from the aforementioned lateral aperture(s).

It is expected that the implements of the invention may also benefit from robotics-mediated procedures, e.g., where the robotics are functionally integrated/interfaced, such as is finding increased use in delicate medical and experimental procedures.

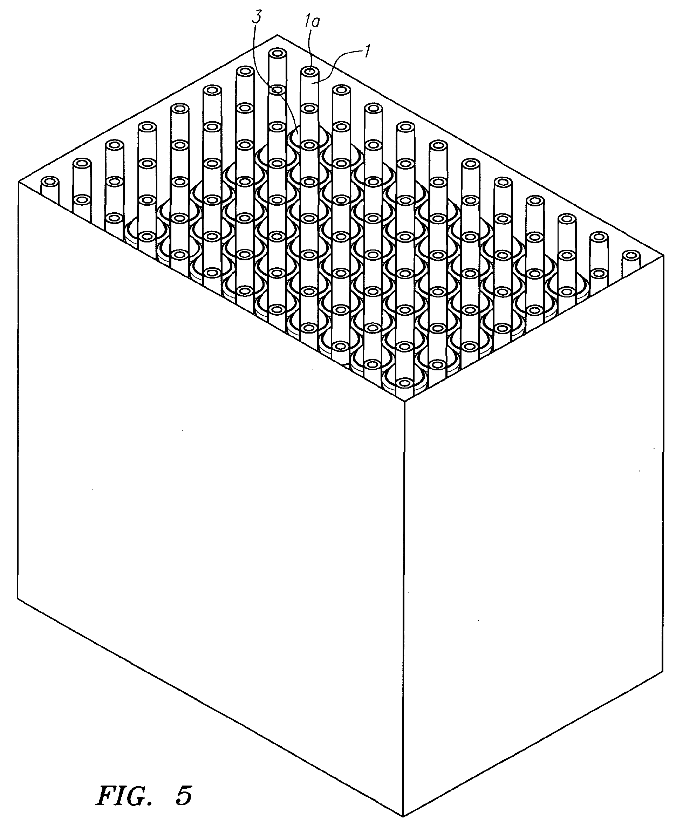

Kits can include multiple individually wrapped implements or boxing about a plurality nested singular implements, each wrapped or unwrapped. Figure 5 provides an example of the latter. As the person of skill will appreciate, there are many variations of kit configuration possible and any given kit can also feature two or more implements of different size, dimension, or color, with those of the same size, dimension and/or color segregated together, e.g., in a different pouch or packaging relative to other dimensioned, sized or colored implements.

The following examples are further illustrative of different aspects and embodiments of the invention.

### EXAMPLES

### EXAMPLE 1: A SINGLE BALL AND SOCKET JOINT EMBODIMENT

Figure 1, including its subparts, shows a two-piece suction implement not forming part of the invention such as claimed, consisting of
a nozzle portion 1 and a handle/body portion **2.** Nozzle portion **1** features a hollow interior luminal space terminating in two open ends: a patient contacting end **1a** and second end **1b** that in this embodiment is cup- or socket-shaped. Handle portion **2** also contains two ends, a source end **2a** for connecting to a vacuum line or pump (not shown) and a second end **2b** configured in this embodiment as ball-shaped to be received within and functionally mated with socket/cup-shaped nozzle end **1b.** Mating permits a rotational and angular movement of the cup about the ball and the fit between the two is preferably frictionally tight. The cup and ball also each have opposing luminal channel orifices that line up when ball is fitted within cup and that permit flow through the overall implement when in use. Also shown is a flanged ring **2c** on handle portion that facilitates sealing with a source or vacuum line (not shown). The ball-shaped male end need not be perfectly spherical or hemispherical as long as rotational ability and luminal space communication during vacuuming/suctioning/irrigation is sufficiently retained for the procedure at hand. The top of the ball, for example, may give the impression of having been planed to allow for a luminal bore or channel there through to interface with a corresponding luminal bore or channel in the complementary female socket. When fitted together the luminal channels abut to communicate with one another along the length of the implement as a whole.

The ball and socket design limits somewhat the maximum angle of bend that can be achieved, but this can be overcome, e.g., by adding one or more additional ball and sockets in series, e.g., as shown in the joint depicted in Figure 2.

The fit between the male and female ends should be frictionally tight but still permit operational rotation or angling upon application of desired force. The male portion can be retained within the female portion.

An internal flanged ring or series of retaining points or detents in or along the inner face of the mouth of the female socket (not shown in the figures) can optionally be used to retain the ball upon snap or press-fitting into the female flanged end, such that the ball can still rotate in the socket, but resist detaching from the socket. This ring or series of retaining points can be integral to the female piece and corresponding mold. The person of ordinary skill will appreciate that other retaining means can also be used.

The ends may be made to be conforming to one another, i.e. one or both ends can expand or compress relative to their complementary end upon press- or snap-fitting to allow the ends to engage one another with the desired functional fit.

### EXAMPLE 2: A DUAL BALL AND SOCKET JOINT EMBODIMENT

Figure 2 shows an implement embodiment according to the invention such as claimed, featuring a dual ball and socket joint in
which the second end of the nozzle portion **1b** does not directly contact the second end of the handle portion **2b,** but does so indirectly via adapter/connector **3** interfaces **3a** and **3b,** which in this particular configuration are essentially identical and inter-convertible with one another. Adapter/connector ends **3a** and **3b** could both equally well be ball-shaped were nozzle portion end **1b** and handle portion end **2b** both configured as socket-shaped. Alternatively, adapter/connector **3** could be configured as asymmetrical, with one end featuring a ball and the other end a socket, each designed to engage a reciprocally configured end of the respective nozzle and handle portions.

Lockwood Products, Inc. of Lake Oswego, OR, USA (*see* www.locline.com/couplings-adaptors.php) offers a variety of different adapter/connector types for other purposes on which the adapter connectors of the instant invention could be based or mimicked in whole or part.

### EXAMPLE 3: A ROTARY JOINT EMBODIMENT

Figures 3 and 4 illustrate two rotary joint embodiments not forming part of the invention such as claimed, in which ends 1b and 2b are
not ball and socket configured and functionally marry at an angle (here ∼90°) relative to the longitudinal axes of the handle and nozzle portions when those portions are extended maximally away from each other. In this particular embodiment, the joint-mating portions of ends **1b** and **2b,** respectively **1bi** and **2bi,** are of complementary design and configuration such that when mated they afford a rotationally communicative drum joint having an internal luminal passageway extending along the entire implement as for the previously discussed embodiments. Such configuration could make use of a shunted or skewed internal luminal spacing at the point of intersection of opposing complementary pieces (e.g., relative to the central longitudinal axis of the handle at the point of intersection with the abutting reciprocal facing supplied by the nozzle, or vice-versa).

Regardless, the degree of transverseness of the rotary joint position relative to handle longitudinal axis can be anywhere from 0 to 90° or more.

The advantage of this particular type of joint is that it can offer greater angular reach of nozzle end **1a** than can the ball and socket joints of Examples 1 and 2. If the transverse joint is configured long/wide enough and perpendicular enough relative to the handle and nozzle portions and their axes, a 360° rotation can theoretically be achieved, as the handle and nozzle portions could be configured to rotate in substantially parallel planes with respect to one another.

In an alternative rotary joint embodiment, the joint could also take on a ball and socket configuration (not shown), with a transverse axial angle relative to the ball and socket joints of Examples/Figures 1 and 2, with the internal passageway/luminal space functionally preserved as for the ball and socket embodiments previously discussed.

The substance and description of all patents cited herein, including all of the references cited therein, are mentioned to further
facilitate an understanding of the background art and demonstrate the level of skill and/or knowledge in the art.

The person of skill will appreciate that the specification is only illustrative and that additional aspects and embodiments will be clear upon study of the specification, drawings and claims.

Also, the transitional claim terms "comprising," "consisting essentially of," and "consisting of" can all be substituted for one another herein, with corresponding varying effect on meaning, and each of these variations can embrace different scopes of the overall inventive aspects.

### INDUSTRIAL APPLICABILITY

The invention relates to implements used daily all around the world, and that are readily capable of mass production to meet that great need, e.g., through simple injection molding. The invention thus has good industrial applicability.

## Claims

1. An adjustable suctioning implement for dental or medical applications, comprising:
a handle portion having a source end diameter of width between about 0.40 and 0.60 inches (1.1016-1.524 cm) for connecting to a vacuum line or pump, and a second end;
a nozzle portion having a contacting end for engaging a patient surface to be suctioned and a second end for functionally engaging, directly or indirectly, said second end of said handle portion,
a dual ball and socket joint portion defined at least in part by said second end of said handle portion and said second end of said nozzle portion;
and an internal hollow conduit extending from said contacting end of said nozzle portion to said
source end of said handle portion and configured to interface with said vacuum line or pump;
said dual ball and socket joint comprises a single modular adapter cooperatively positioned between and abutting said second end of said nozzle portion and said second end of said handle portion.

2. The adjustable suctioning implement of claim 1 wherein said modular adapter comprises either two integrally molded balls or two integrally molded sockets facing away from each other.

3. The adjustable suctioning implement of claim 1 further comprising an aperture in one or more of said handle portion, nozzle portion or joint, said aperture in communication with said conduit and allowing for operator-mediated pressure adjustment at the surface of engagement.

4. The adjustable suctioning implement of claim 1 comprising one or more materials selected from the group consisting of metal, molded plastic, rubber, glass and paper.

5. The adjustable suctioning implement of claim 1 comprising, at least in part, injection molded plastic.

6. The adjustable suctioning implement of claim 1 wherein at least one of said handle and said nozzle portions features a tapering from one end of said portion to the other end of said portion.

7. The adjustable suctioning implement of claim 1 having one or more dimensions selected from the range of dimensions consisting of:
nozzle portion length: 0.1-5.0 inches (0.254- 12.7 cm);
nozzle portion width: 0.1-0.5 inches (0.254-1.27 cm);
handle portion length: 1.5-6.0 inches (3.81-15.24cm);
handle portion width: 0.1-1.0 inches (0.254-2.54 cm);
and wall thickness: 0.01-0.10 inches (0.0254-0.254 cm).

8. The adjustable suctioning implement of claim 1 wherein said contacting end of said nozzle portion is beveled.

9. The adjustable suctioning implement of claim 1 wherein said contacting end of said nozzle portion is not
perpendicular to the longitudinal plane of said nozzle. portion 12

10. The adjustable suctioning implement of claim 1 further comprising a screen to capture debris.

11. A kit comprising a plurality of adjustable suctioning implements according to claim 7.

12. The kit of claim 11 wherein said plurality of adjustable suctioning implements are individually wrapped.

13. The kit of claim 11 wherein said plurality of adjustable suctioning implements comprise two or more
implements of different size, dimension, or color.

14. The kit of claim 13 comprising adjustable suctioning implements of at least two different dimensions selected
from the range of dimensions listed in claim 7.

15. A method of using the adjustable suctioning implement of claim 1, comprising: connecting a pump or
aspirator to the source end of the handle portion of the implement of any of claims 1- 10; and
applying the contacting end of the nozzle portion of said implement to a surface to be suctioned.

16. The method of claim 15 wherein said method is operated during a dental or oral surgery procedure.

17. The method of claim 15wherein said method is operated during a medical procedure other than dentistry or oral surgery.

18. The method of claim 15 wherein said method is operated during a medical procedure other than
dental.

## Patentansprüche

1. Einstellbares Absauggerät für dentale oder medizinische Anwendungen, umfassend:
einen Griffabschnitt mit einem Ausgangsenddurchmesser einer Breite zwischen ungefähr 0,40 und 0,60 Zoll (1,1016 bis 1,524 cm) zum Anschließen einer Unterdruckleitung oder -pumpe und einem zweiten Ende;
einen Düsenabschnitt mit einem Kontaktende zum Eingreifen in eine abzusaugende Patientenfläche und einem zweiten Ende zum funktionalen Eingreifen direkt oder indirekt in das zweite Ende des genannten Griffabschnitts,
eine Doppelkugel und einen Fassungsgelenkabschnitt, der wenigstens teilweise durch das genannte zweite Ende des genannten Griffabschnitts und des genannten zweiten Endes des genannten Düsenabschnitts definiert ist;
und eine interne Hohlleitung, die sich von dem genannten Kontaktende des genannten Düsenabschnitts zu dem genannten Ausgangsende des genannten Griffabschnitts erstreckt und konfiguriert ist, um mit der genannten Unterdruckleitung oder - pumpe eine Schnittstelle zu bilden;
die genannte Doppelkugel und das Fassungsgelenk umfassen einen einzelnen, modularen Adapter, der zusammenwirkend zwischen dem genannten zweiten Ende des genannten Düsenabschnitts und dem genannten zweiten Ende des genannten Griffabschnitts und daran angrenzend positioniert ist.

2. Einstellbares Absauggerät gemäß Anspruch 1, wobei der modulare Adapter entweder zwei aufgepresste Kugeln oder zwei aufgepresste Fassungen umfasst, die voneinander weg angeordnet sind.

3. Einstellbares Absauggerät gemäß Anspruch 1, weiterhin umfassend eine Öffnung in einem mehr mehreren des genannten Griffabschnitts, Düsenabschnitts oder Gelenk, wobei die genannte Öffnung mit der genannten Leitung in Verbindung steht und eine operatorvermittelte Druckanpassung an der Eingreiffläche zulässt.

4. Einstellbares Absauggerät gemäß Anspruch 1, umfassend ein oder mehrere Materialien, die aus der Gruppe ausgewählt sind, bestehend aus Metall, abgeformtem Plastik, Gummi, Glas und Papier.

5. Einstellbares Absauggerät gemäß Anspruch 1, umfassend wenigstens teilweise spritzgeformtes Plastik.

6. Einstellbares Absauggerät gemäß Anspruch 1, wobei wenigstens einer der genannten Griff- und der genannten Düsenabschnitte eine Verjüngung von einem Ende des genannten Abschnitts zum anderen Ende des genannten Abschnitts aufweist.

7. Einstellbares Absauggerät gemäß Anspruch 1, wobei eine oder mehrere Abmessungen aus dem Abmessungsbereich ausgewählt ist, bestehend aus:
Düsenabschnitt Länge: 0,1 bis 5,0 Zoll (0,254 bis 12,7 cm);
Düsenabschnitt Breite:0,1 bis 0,5 Zoll (0,254 bis 1,27 cm);
Griffabschnitt Länge: 1,5 bis 6,0 Zoll (3,81 bis 15,24 cm);
Griffabschnitt Breite: 0,1 bis 1,0 Zoll (0,254 bis 2,54 cm);
und Wanddicke; 0,01 bis 0,10 Zoll (0,0254 bis 0,254 cm).

8. Einstellbares Absauggerät gemäß Anspruch 1, wobei das genannte Kontaktende des genannten Düsenabschnitts abgefast ist.

9. Einstellbares Absauggerät gemäß Anspruch 1, wobei das genannte Kontaktende des genannten Düsenabschnitts nicht lotrecht zu der Längsebene des genannten Düsenabschnitts ist.

10. Einstellbares Absauggerät gemäß Anspruch 1, weiterhin umfassend ein Sieb zum Auffangen von Fremdkörpern.

11. Kit, umfassend eine Vielzahl von einstellbaren Absauggeräten gemäß Anspruch 7.

12. Kit gemäß Anspruch 11, wobei die genannte Vielzahl von einstellbaren Absauggeräten jeweils einzeln verpackt ist.

13. Kit gemäß Anspruch 11, wobei die genannte Vielzahl von einstellbaren Absauggeräten zwei oder mehr Geräte unterschiedlicher Größe, Abmessung oder Farbe umfasst.

14. Kit gemäß Anspruch 13, umfassend einstellbare Absauggeräte wenigstens zwei unterschiedlicher Abmessungen, die ausgewählt sind aus dem in Anspruch 7 aufgelisteten Abmessungsbereich.

15. Verfahren zur Verwendung des einstellbaren Absauggeräts gemäß Anspruch 1, umfassend: Anschließen einer Pumpe oder einer Absaugung an das Ausgangsende des Griffabschnitts des Geräts gemäß Anspruch 1 bis 10; und
Anwenden des Kontaktendes des Düsenabschnitts des genannten Geräts auf eine abzusaugende Fläche.

16. Verfahren gemäß Anspruch 15, wobei das genannte Verfahren während eines dentalen oder oralen Operationsprozesses angewendet wird.

17. Verfahren gemäß Anspruch 15, wobei das genannte Verfahren während eines medizinischen Verfahrens mit Ausnahme einer dentalen oder oralen Operation angewendet wird.

18. Verfahren gemäß Anspruch 15, wobei das genannte Verfahren während eines medizinischen Verfahrens mit Ausnahme eines dentalen Verfahrens angewendet wird.

## Revendications

1. Outil d'aspiration ajustable pour utilisations dentaires ou médicales, comprenant:
un manche ayant une extrémité de source dont le diamètre est compris entre 0,40 et 0,60 Pouces (1,1016 à 1,524 cm) pour être raccordée à une conduite ou pompe à vide, et une seconde extrémité ;
une buse ayant une extrémité de contact pour s'engager sur une surface de patient à aspirer et une seconde extrémité pour s'engager en fonctionnement, directement ou indirectement, sur ladite seconde extrémité dudit manche ;
une articulation à double rotule au moins partiellement définie par ladite seconde extrémité dudit manche et ladite seconde extrémité de ladite buse ;
et un conduit creux interne s'étendant entre ladite extrémité de contact de ladite buse et ladite extrémité de source dudit manche et configuré pour s'interfacer avec ladite conduite ou pompe à vide ;
ladite articulation à double rotule comprend un adaptateur modulaire unique positionné en coopération entre et en appui sur ladite seconde extrémité de ladite buse et ladite seconde extrémité dudit manche.

2. Outil d'aspiration ajustable selon la revendication 1, dans lequel ledit adaptateur modulaire comprend soit deux boules moulées en une seule pièce soit deux cavités moulées en une seule pièce se faisant face à l'opposé l'une de l'autre.

3. Outil d'aspiration ajustable selon la revendication 1, comprenant, en outre, une ouverture dans un ou plusieurs dudit manche, de ladite buse ou articulation, ladite ouverture étant en communication avec ledit conduit et permettant de régler la pression de l'opérateur à la surface d'engagement.

4. Outil d'aspiration ajustable selon la revendication 1, comprenant un ou plusieurs matériaux choisis dans le groupe consistant en métal, plastique moulé, caoutchouc, verre et papier.

5. Outil d'aspiration ajustable selon la revendication 1, comprenant, au moins partiellement, un plastique moulé par injection.

6. Outil d'aspiration ajustable selon la revendication 1, dans lequel au moins un dudit manche et de ladite buse présente une partie effilée d'une extrémité de ladite partie à l'autre extrémité de ladite partie.

7. Outil d'aspiration ajustable selon la revendication 1, ayant une ou plusieurs dimensions choisis dans la plage de dimensions consistant en :
longueur de buse : 0,1 à 5,0 Pouces (0,254 à 12,7 cm) ;
largeur de buse : 0,1 à 0,5 Pouces (0,254 à 1,27 cm);
longueur de manche : 1,5 à 6,0 Pouces (3,81 à 15,24 cm) ;
largeur de manche : 0,1 à 1,0 Pouces (0,254 à 2,54 cm);
et épaisseur de paroi : 0,01 à 0,10 Pouces (0,0254 à 0,254 cm).

8. Outil d'aspiration ajustable selon la revendication 1, dans lequel ladite extrémité de contact de ladite buse est en biseau.

9. Outil d'aspiration ajustable selon la revendication 1, dans lequel ladite extrémité de contact de ladite buse n'est pas perpendiculaire au plan longitudinal de ladite buse.

10. Outil d'aspiration ajustable selon la revendication 1, comprenant, en outre, un tamis pour collecter les débris.

11. Kit comprenant une pluralité d'outils d'aspiration ajustables selon la revendication 7.

12. Kit selon la revendication 11, dans lequel ladite pluralité d'outils d'aspiration ajustables est conditionnée séparément.

13. Kit selon la revendication 11, dans lequel ladite pluralité d'outils d'aspiration ajustables comprend deux outils ou plus de taille, dimension ou couleur différentes.

14. Kit selon la revendication 13, comprenant des outils d'aspiration ajustables d'au moins deux dimensions différentes choisies dans la plage de dimensions énumérées dans la revendication 7.

15. Procédé d'utilisation de l'outil d'aspiration ajustable selon la revendication 1, comprenant: le raccordement d'une pompe ou d'un aspirateur à l'extrémité de la source du manche de l'outil selon l'une quelconque des revendications 1 à 10 ; et
l'application de l'extrémité de contact de la buse dudit outil sur une surface à aspirer.

16. Procédé selon la revendication 15, dans lequel ledit procédé est actionné pendant une procédure chirurgicale dentaire ou orale.

17. Procédé selon la revendication 15, dans lequel ledit procédé est actionné pendant une procédure médicale, autre que de chirurgie dentaire ou orale.

18. Procédé selon la revendication 15, dans lequel ledit procédé est actionné pendant une procédure chirurgicale, autre que dentaire.
